# EUROPEAN PATENT APPLICATION

(11) **EP 3 789 492 A1**
(43) Date of publication of application: **10.03.2021**
(21) Application number: 19195522.8
(22) Date of filing: 05.09.2019
(51) Int. Cl.: C12N 15/63, C12Q 1/6897, G01N 33/68

(54) **FLUORESCENCE-BASED CELLULAR ASSAY FOR THE DETECTION OF GENE-REGULATORY ACTIVITY OF CHROMATIN-ASSOCIATED PROTEINS AND PROTEIN COMPLEXES**

(71) Applicant: Universität Stuttgart, 70174 Stuttgart (DE)
(72) Inventor: RATHERT, Philipp, 70569 Stuttgart (DE); PINTER, Sabine, 70569 Stuttgart (DE)
(74) Representative: Müller-Boré & Partner Patentanwälte PartG mbB

(57) **Abstract**

The present invention relates to methods for the identification of co-regulatory factors that modulate the activity of (i) a chromatin-associated protein, and/or (ii) a binding partner of said chromatin-associated protein, and/or (iii) a factor that modulates the activity of said chromatin-associated protein without forming a physically stable complex with the same, in a cell.

## Description

The present invention relates to methods for the identification of co-regulatory factors that modulate the activity of (i) a chromatin-associated protein, and/or (ii) a binding partner of said chromatin-associated protein, and/or (iii) a factor that modulates the activity of said chromatin-associated protein without forming a physically stable complex with the same, in a cell.

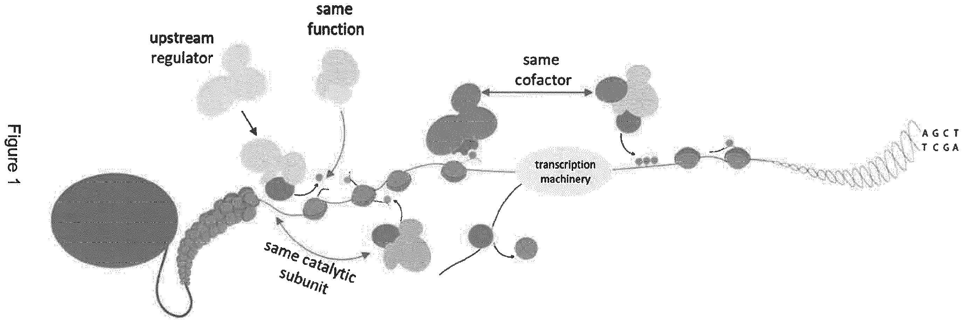

DNA gene expression in eukaryotic cells is regulated by epigenetic modifications that mediate communication between the environment and the genome. These modifications include DNA methylation and posttranslational modifications of histones, such as methylation, acetylation or ubiquitination, which affect gene expression independently of the DNA sequence. Epigenetic modifications have either a repressive or activating effect on gene expression and are placed, recognized/read, stabilized and removed by various chromatin-associated epigenetic effector proteins (Fig. 1). Essential processes such as gene expression, DNA repair and recombination are regulated by these chromatin-associated proteins.

Epigenetic mechanisms are crucial for the development of different cell types and the maintenance of a healthy cell. Faulty epigenetic states, e.g. caused by mutations in chromatin regulators, can influence key cell processes that ultimately lead to oncogenic transformation and tumor development. In contrast to the majority of mutations in tumor suppressors and oncogenes, epigenetic effector proteins are accessible to drug therapy and thus attractive targets for drug development. In October 2016, a total of 112 compounds were in clinical development. Approximately 25% of these compounds were in Phase II and III clinical trials. A total of seven active substances against epigenetic effectors for the treatment of a wide variety of leukemias were in clinical application in 2016.

One challenge in research, as well as for the development of epigenetic therapies, is to identify and characterize these epigenetic (epi)-effector proteins and (co-)regulators of the same, and to develop efficient inhibitors. The difficulty lies in the fact that epigenetic effector proteins often combine different functions, e.g. contain several reading and writing domains for different epigenetic marks (Fig. 1). In addition, epigenetic effector proteins generally recruit a large number of other proteins and form large multiprotein complexes with them, most of which have not yet been investigated in their entirety (Fig. 1). The function of each individual protein of the complex on gene expression is largely unknown. In addition, epigenetic effector proteins and complexes can regulate gene expression by inhibiting or activating other proteins and complexes (Fig. 1). Moreover, epigenetic effector proteins can be part of more than one complex at a time, and complexes themselves can be the subject of regulation by a wide range of (co-)regulatory factors (Fig. 1). This makes the entire epigenetic network highly complex and difficult to study. Inhibitors of an epigenetic effector may be effective in biochemical assays, but ineffective in the totality of the multiprotein complex, since much of the influence on gene regulatory activity can be mediated by complex partners.

So far, attempts have been made to uncover complex partners of epigenetic effectors by co-immunoprecipitation (Co-IP) followed by Western blot or mass spectrometry (MS) analyses. Drug research is limited to *in vitro* assays with purified epigenetic effector proteins or minimal complexes with two or three complex subunits and mass spectrometric analysis of epigenetic multiprotein complexes after purification via subunit-specific inhibitors (so-called "chemoproteomics").

The approaches to characterize epigenetic multiprotein complexes by immuno-purification and subsequent analysis via Western blot or MS analysis are complex and require specific antibodies. In addition, only complex partners are identified that are very abundant and interact extremely stable with the investigated epi-effector protein. In addition, these approaches do not provide information on functional dependencies.

Inhibitors identified via *in vitro* assays with purified epi-effectors or minimal complexes often turn out to be ineffective in cellular assays because the interaction of the epi-effector with its complex partners with other regulatory protein subunits was not considered in the drug identification.

Chemoproteomics considers these complexes but does not provide any information on the influence of the complex subunits on the function of the complex.

In view of the complexity of epigenetic mechanisms and their regulation, it is necessary to develop biological tools that significantly increase the level of knowledge in order to develop more efficient therapeutic approaches. This requires the development of reporter systems that allow investigation of the specific function of epigenetic proteins and directly analysis of the complex regulatory network.

Accordingly, the technical problem underlying the present invention is the provision of means for the detection of co-regulatory factors that modulate the activity of a chromatin-associated protein, and/or a binding partner of said chromatin-associated protein, and/or a factor that modulates the activity of said chromatin-associated protein without forming a physically stable complex with the same, in a cell.

The solution to the above technical problem is achieved by the embodiments characterized in the claims.

In particular, in a first aspect, the present invention relates to a method for the identification of a co-regulatory factor that modulates the activity of (i) a chromatin-associated protein and/or (ii) a binding partner of said chromatin-associated protein, and/or (iii) a factor that modulates the activity of said chromatin-associated protein without forming a physically stable complex with the same, in a cell, comprising the steps of:
(a) providing a cell that
   (i) expresses a fusion protein of said chromatin-associated protein with either a reverse tetracycline-controlled transactivator protein (rtTA) or a tetracycline-controlled transactivator protein (tTA), and
   (ii) is capable of expressing a reporter protein under control of a tetracycline response element (TRE);
(b) modulating the expression of a factor of interest in said cell;
(c) providing a tetracycline to the cell in amount sufficient to activate rtTA or tTA while the modulation of the expression of said factor of interest persists;
(d) detecting any modulation of the expression of said reporter protein in said cell;
(e) identifying the factor of interest as a co-regulatory factor that modulates the activity of said chromatin-associated protein and/or a binding partner of said chromatin-associated protein, and/or a factor that modulates the activity of said chromatin-associated protein without forming a physically stable complex with the same, in case the modulation of the expression of said reporter protein detected in step (d) differs from the modulation of the expression of said reporter protein detected in a control cell in which the expression of said factor of interest has not been modulated.

The methods of the present invention will identify a co-regulatory factor, preferably a co-regulatory factor that is a protein, that modulates the activity of (i) a chromatin-associated protein, and/or (ii) a binding partner of said chromatin-associated protein, and/or (iii) a factor that modulates the activity of said chromatin-associated protein without forming a physically stable complex with the same, in a cell. However, in preferred embodiments, said co-regulatory factor is not a strong physical complex partner of said chromatin-associated protein, or of a complex partner of said chromatin-associated protein, or is not a complex partner of said chromatin-associated protein, or of a complex partner of said chromatin-associated protein, at all. Due to the methodical design, identification of a co-regulatory factor with this method is conditional on a merely functional connection between co-regulatory factor and chromatin-associated protein. This will allow to detect novel functional dependencies in epigenetic networks, compared to screening methods that depend on the temporal and chemical stability of the physical complex of two or more proteins.

As used herein, the term "modulates/modulation" refers to any up- or down-regulation of the modulated parameter.

Further, as used herein, the term "co-regulatory factor" refers to any factor, preferably protein factor, that directly or indirectly modulates the activity of its target. In this context, indirect modulation refers to a modulation with one or more intermediate factors that exert a regulatory function in a regulatory pathway starting at the co-regulatory factor, extending to the one or more intermediate factors, and ending at its target.

Furthermore, as used herein, the term "activity" refers to enzymatic activity, but can also refer to a proteins' capability of binding to a binding partner or the proteins' ability to modulate gene expression.

As used herein, the term "chromatin-associated protein" refers to any protein that in some form directly or indirectly interacts with chromatin in a living cell. In many cases, chromatin-associated proteins form complexes, i.e., multiprotein complexes, with one or more complex partners, wherein a given chromatin-associated protein can be part of more than one particular protein complex. Chromatin-associated proteins that can be subject of the methods of the present invention are not particularly limited and are known in the art. In preferred embodiments, the chromatin-associated protein is an epigenetic effector protein, *i.e.,* a protein that in some form mediates heritable gene activity and/or gene expression phenotypes. Respective epigenetic effector proteins are not particularly limited and are known in the art. Specific examples of epigenetic effector proteins include Lysine-specific histone demethylase 1A (LSD1), Euchromatic Histone Lysine Methyltransferase 1 (EHMT1), DNA Methyltransferase 3 Alpha (DNMT3A) and Embryonic Ectoderm Development (EED).

Further examples of epigenetic effector proteins in the context of the present invention include the proteins ACIN1, ACTA1, ACTB, ACTL6A, ACTL6B, ACTR3B, ACTR5, ACTR6, ACTR8, ADA, ADAR, ADPRH, ADPRHL2, AEBP2, AFF1, AFF4, AICDA, AIRE, AKAP1, AKAP8, ALKBH2, ALKBH3, ALKBH8, AP2A1, APBB1, APITD1, APOBEC2, APOBEC3B, APOBEC3C, APOBEC3D, APOBEC3F, APOBEC3G, APOBEC3H, APOBEC4, ARID1A, ARID1B, ARID2, ARID4A, ARID4B, ARIH2, ART1, AS3MT, ASAP1, ASCC3, ASCL2, ASF1A, ASF1B, ASH1L, ASH2L, ASMT, ASXL1, ASXL2, ASXL3, ATAD2, ATAD2B, ATAT1, ATF2, ATF7IP, ATF7IP2, ATM, ATOH1, ATR, ATRX, ATXN3, ATXN7, ATXN7L3, AURKA, AURKB, BABAM1, BAHCC1, BAHD1, BANP, BAP1, BAZ1A, BAZ1B, BAZ2A, BAZ2B, BCL6, BCOR, BCORL1, BDP1, BLM, BMI1, BOP1, BPTF, BRCA1, BRCA2, BRCC3, BRD1, BRD2, BRD3, BRD4, BRD7, BRD8, BRD9, BRDT, BRE, BRIP1, BRPF1, BRPF3, BRWD1, BRWD3, BTAF1, BUB3, C11orf30, C14orf169, C14orf43, C14orf93, C16orf53, C17orf49, C17orf96, C20orf20, C6orf130, CALCOCO1, CAMK4, CAMKMT, CARM1, CBL, CBX1, CBX2, CBX3, CBX4, CBX5, CBX6, CBX7, CBX8, CCDC101, CCDC79, CDA, CDC34, CDC5L, CDC6, CDCA2, CDCA7L, CDK17, CDK2AP1, CDK3, CDK5, CDK9, CDY1, CDY2A, CDYL, CDYL2, CECR1, CECR2, CENPA, CENPB, CENPC1, CENPE, CENPF, CENPH, CENPI, CENPJ, CENPK, CENPL, CENPM, CENPN, CENPO, CENPP, CENPQ, CENPT, CENPV, CENPW, CHAF1A, CHAF1B, CHD1, CHD1L, CHD2, CHD3, CHD4, CHD5, CHD6, CHD7, CHD8, CHD9, CHMP1A, CHMP1B, CHMP2A, CHMP2B, CHMP3, CHMP4A, CHMP4B, CHMP4C, CHMP5, CHMP6, CHMP7, CHRAC1, CHTOP, CHUK, CLOCK, COMMD3-BMI1, COQ3, COQ5, CRAMP1L, CREBBP, CSF2RA, CSNK2A1, CSNK2B, CSTL1, CTCF, CTSL1, CTSL2, CUX1, CXXC1, CXXC4, CXXC5, DAPK3, DAXX, DCTD, DDB1, DDX1, DDX10, DDX11, DDX17, DDX18, DDX19A, DDX19B, DDX20, DDX21, DDX23, DDX24, DDX25, DDX27, DDX28, DDX31, DDX39A, DDX39B, DDX3X, DDX3Y, DDX4, DDX41, DDX42, DDX43, DDX46, DDX47, DDX49, DDX5, DDX50, DDX51, DDX52, DDX53, DDX54, DDX55, DDX56, DDX58, DDX59, DDX6, DDX60, DDX60L, DHX15, DHX16, DHX29, DHX30, DHX32, DHX33, DHX34, DHX35, DHX36, DHX37, DHX38, DHX40, DHX57, DHX58, DHX8, DHX9, DIDO1, DMAP1, DMTF1, DNA2, DNAJC1, DNAJC2, DNMT1, DNMT3A, DNMT3B, DNMT3L, DOT1L, DPF1, DPF2, DPF3, DPY30, DQX1, DSN1, DTX3L, DZIP3, ECE2, EDF1, EED, EHMT1, EHMT2, EID1, EIF4A1, EIF4A2, EIF4A3, ELP2, ELP3, ELP4, EMG1, ENY2, EP300, EP400, EPC1, EPC2, ERCC2, ERCC3, ERCC5, ERCC6, ERCC6L, ERCC6L2, ERG, ERI1, ESCO1, ESCO2, EYA1, EYA2, EZH1, EZH2, FABP1, FAM175A, FAM175B, FAM208A, FANCM, FBXL19, FBXO10, FBXO11, FBXO17, FBXO44, FBXW9, FEN1, FIZ1, FKBP1A, FKBP2, FKBP5, FLYWCH1, FMR1, FOXA1, FOXA2, FOXA3, FOXO1, FOXO3, FOXO4, FOXP1, FOXP2, FOXP3, FOXP4, FTSJ3, FUBP3, FUS, FXR1, FXR2, G2E3, GADD45A, GADD45B, GATAD2A, GATAD2B, GDAP2, GFI1B, GLI1, GLI3, GLYR1, GON4L, GPS2, GSG2, GTF2B, GTF2F1, GTF2H1, GTF3C4, GTF3C5, H1FO, H1FNT, H1FOO, H1FX, H2AFB3, H2AFJ, H2AFV, H2AFX, H2AFY, H2AFY2, H2AFZ, H2BFM, H2BFWT, H3F3A, H3F3B, H3F3C, HAT1, HCFC1, HDAC1, HDAC10, HDAC11, HDAC2, HDAC3, HDAC4, HDAC5, HDAC6, HDAC7, HDAC8, HDAC9, HDGF, HDGFL1, HDGFRP2, HDGFRP3, HELLS, HELQ, HFM1, HIF1AN, HINFP, HIRA, HIST1H1A, HIST1H1B, HIST1H1C, HIST1H1D, HIST1H1E, HIST1H1T, HIST1H2AA, HIST1H2AB, HIST1H2AC, HIST1H2AD, HIST1H2AE, HIST1H2AG, HIST1H2AH, HIST1H2AI, HIST1H2AJ, HIST1H2AK, HIST1H2AL, HIST1H2AM, HIST1H2BA, HIST1H2BB, HIST1H2BC, HIST1H2BD, HIST1H2BE, HIST1H2BF, HIST1H2BG, HIST1H2BH, HIST1H2BI, HIST1H2BJ, HIST1H2BK, HIST1H2BL, HIST1H2BM, HIST1H2BN, HIST1H2BO, HIST1H3A, HIST1H3B, HIST1H3C, HIST1H3D, HIST1H3E, HIST1H3F, HIST1H3G, HIST1H3H, HIST1H3I, HIST1H3J, HIST1H4A, HIST1H4B, HIST1H4C, HIST1H4D, HIST1H4E, HIST1H4F, HIST1H4G, HIST1H4H, HIST1H4I, HIST1H4J, HIST1H4K, HIST1H4L, HIST2H2AA3, HIST2H2AB, HIST2H2AC, HIST2H2BE, HIST2H2BF, HIST2H3C, HIST2H3D, HIST2H4A, HIST3H2A, HIST3H2BB, HIST3H3, HIST4H4, HJURP, HLCS, HLTF, HMG20A, HMG20B, HMGA1, HMGA2, HMGN1, HMGN2, HMGN3, HMGN4, HMGN5, HNRNPA0, HNRNPA1, HNRNPA2B1, HOMEZ, HOXC11, HP1BP3, HPRT1, HR, HSPBAP1, HUWE1, IFIH1, IGHMBP2, IKBKAP, ING1, ING2, ING3, ING4, ING5, INMT, INO80, INO80B, INO80C, INO80D, INO80E, INTS12, IWS1, JAK2, JARID2, JHDM1D, JMJD1C, JMJD4, JMJD6, JMJD7, JMJD7-PLA2G4B, JMJD8, JUNB, KANSL1, KAT2A, KAT2B, KAT5, KAT6A, KAT6B, KAT7, KAT8, KDM1A, KDM1B, KDM2A, KDM2B, KDM3A, KDM3B, KDM4A, KDM4B, KDM4C, KDM4D, KDM4E, KDM5A, KDM5B, KDM5C, KDM5D, KDM6A, KDM6B, KDM8, KEAP1, KIAA1045, KIAA1958, KIAA2026, KIF22, KLF1, KLHDC3, KNTC1, L3MBTL1, L3MBTL2, L3MBTL3, L3MBTL4, LBR, LCOR, LMNA, LMNB1, LMNB2, LNX1, LRWD1, MACROD1, MACROD2, MAEL, MAP3K12, MAU2, MAZ, MBD1, MBD2, MBD3, MBD4, MBD5, MBD6, MBTD1, MCM2, MCM3, MCM4, MCM5, MCM6, MCM7, MCRS1, MDC1, MEAF6, MECOM, MECP2, MED1, MEN1, METTL10, METTL11B, METTL12, METTL13, METTL20, METTL21D, METTL2A, METTL2B, METTL6, METTL7A, METTL7B, METTL8, MGEA5, MGMT, MIER1, MIER2, MIER3, MINA, MIS12, MIS18A, MIS18BP1, MKRN1, MLH3, MLL, MLL2, MLL3, MLL4, MLL5, MLLT10, MLLT6, MORC1, MORC2, MORC3, MORC4, MORF4L1, MORF4L2, MPHOSPH8, MPL, MSH6, MSL2, MSL3, MST1, MTA1, MTA2, MTA3, MTERF, MTF1, MTF2, MTR, MUL1, MUM1, MUM1L1, MYB, MYBL1, MYBL2, MYC, MYPOP, MYSM1, NAA10, NAA15, NAA60, NAP1L1, NAP1L2, NAP1L3, NAT10, NAT6, NBN, NCOA1, NCOA3, NCOA6, NCOR1, NCOR2, NDC80, NDUFAF5, NEDD4L, NEK6, NEK9, NFE2, NFRKB, NFYA, NFYB, NFYC, NNMT, NOC2L, NONO, NOP2, NR0B2, NR2C1, NSD1, NSL1, NSMCE2, NSUN2, NSUN3, NSUN4, NSUN5, NSUN6, NSUN7, NTMT1, NUF2, NUFIP1, OGT, OIP5, ORC1, ORC2, PADI1, PADI2, PADI3, PADI4, PADI6, PAF1, PAG1, PAK2, PARG, PARP1, PARP10, PARP11, PARP12, PARP14, PARP15, PARP16, PARP2, PARP3, PARP4, PARP6, PARP8, PARP9, PAX5, PAX6, PAX9, PAXIP1, PBRM1, PBX1, PCGF1, PCGF2, PCGF5, PCGF6, PCMT1, PCNA, PHC1, PHC2, PHC3, PHF1, PHF10, PHF11, PHF12, PHF13, PHF14, PHF15, PHF16, PHF17, PHF19, PHF2, PHF20, PHF20L1, PHF21A, PHF21B, PHF23, PHF3, PHF5A, PHF6, PHF7, PHF8, PHIP, PHRF1, PIAS1, PIAS2, PIAS3, PIAS4, PKN1, PKNOX1, PNMT, POGZ, POLE3, POLQ, POLR1B, POLR2B, POP7, PPARGC1A, PPHLN1, PPIB, PPP4C, PPP5C, PRDM1, PRDM10, PRDM11, PRDM12, PRDM13, PRDM14, PRDM15, PRDM16, PRDM2, PRDM4, PRDM5, PRDM6, PRDM7, PRDM8, PRDM9, PRKAA1, PRKAA2, PRKCB, PRKCD, PRKDC, PRM2, PRMT1, PRMT10, PRMT2, PRMT3, PRMT5, PRMT6, PRMT7, PRMT8, PRTFDC1, PSD3, PSIP1, PWWP2B, PYGO1, PYGO2, RAD18, RAD51, RAD54B, RAD54L, RAD54L2, RAG1, RAG2, RAI1, RANBP2, RASD2, RB1, RBBP4, RBBP5, RBBP7, RBL1, RBL2, RC3H1, RCBTB1, RCOR1, RCOR2, RCOR3, REC8, RECQL, RECQL4, RECQL5, RERE, REST, RFWD2, RFX4, RFX5, RFXANK, RFXAP, RING1, RNF168, RNF17, RNF2, RNF20, RNF217, RNF40, RNF8, RPA1, RPA3, RPH3A, RPP25, RPP25L, RPS19BP1, RPS6KA3, RPS6KA4, RPS6KA5, RSF1, RTEL1, RUNX1, RUVBL1, RUVBL2, SAE1, SAP130, SAP18, SAP25, SAP30, SAP30L, SATB1, SBNO1, SCMH1, SCML2, SCML4, SENP1, SENP2, SENP3, SENP5, SENP6, SENP7, SENP8, SERPINE1, SET, SETD1A, SETD1B, SETD2, SETD3, SETD4, SETD5, SETD6, SETD7, SETD8, SETDB1, SETDB2, SETMAR, SF3B1, SFMBT1, SFMBT2, SHPRH, SIAH2, SIK1, SIN3A, SIN3B, SIRT1, SIRT2, SIRT3, SIRT4, SIRT5, SIRT6, SIRT7, SKA1, SKA2, SKA3, SKIV2L, SKIV2L2, SLBP, SLK, SMARCA1, SMARCA2, SMARCA4, SMARCA5, SMARCAD1, SMARCAL1, SMARCB1, SMARCC1, SMARCC2, SMARCD1, SMARCD2, SMARCD3, SMARCE1, SMC1A, SMC1B, SMN1, SMNDC1, SMYD1, SMYD2, SMYD3, SMYD4, SMYD5, SNAPC4, SND1, SNRNP200, SOX10, SOX15, SP100, SP110, SP140, SP140L, SPC24, SPC25, SRC, SRCAP, SSRP1, STAG1, STAG2, STAG3, STAT5B, STK31, STK4, STRA13, SUB1, SUDS3, SUMO1, SUMO2, SUMO3, SUMO4, SUPT16H, SUPT3H, SUPT7L, SUPV3L1, SUV39H1, SUV39H2, SUV420H1, SUV420H2, SUZ12, SVEP1, TADA1, TADA2A, TADA2B, TADA3, TAF1, TAF10, TAF12, TAF1L, TAF3, TAF5L, TAF6L, TAF9, TAF9B, TBL1X, TBL1XR1, TBL1Y, TCEA1, TCF19, TCF20, TCP1, TDG, TDRD1, TDRD10, TDRD12, TDRD3, TDRD5, TDRD6, TDRD7, TDRD9, TDRKH, TERF1, TERF2, TET1, TET2, TET3, TFPT, TIPARP, TLE1, TLE4, TLK1, TLK2, TMPO, TNKS, TNKS2, TNRC18, TOPORS, TOX4, TP53BP1, TRAF7, TRDMT1, TRERF1, TRIM13, TRIM24, TRIM28, TRIM33, TRIM37, TRIM5, TRIM66, TRRAP, TSPYL2, TSSK6, TTF1, TTF2, UBA1, UBA2, UBA7, UBE2A, UBE2B, UBE2C, UBE2E1, UBE2E3, UBE2I, UBE2M, UBE2N, UBE2Q2, UBE3A, UBE4A, UBR1, UBR2, UBR3, UBR4, UBR5, UBR7, UCHL5, UHRF1, UHRF2, UIMC1, UNG, USF2, USP12, USP13, USP16, USP21, USP22, USP27X, USP3, USP34, USP35, USP46, USP51, USP7, UTP3, UTY, VPS72, VRK1, VRK2, WBSCR22, WBSCR27, WDR5, WDR61, WDR77, WDR82, WHSC1, WHSC1L1, WIZ, WNT5A, WRN, YBX2, YEATS4, YTHDC2, YWHAQ, YY1, ZAK, ZAR1, ZBED5, ZBTB12, ZBTB33, ZBTB38, ZBTB4, ZBTB40, ZBTB9, ZC3HAV1, ZCWPW1, ZCWPW2, ZFP57, ZGPAT, ZHX1, ZHX2, ZHX3, ZKSCAN2, ZMYM1, ZMYM2, ZMYM3, ZMYM4, ZMYM5, ZMYM6, ZMYND11, ZMYND8, ZNF295, ZNF451, ZNF519, ZNF541, ZNF740, ZNF787, ZRANB3, ZSCAN20, ZSCAN21, ZW10, ZWILCH, and ZZZ3, as known in the art.

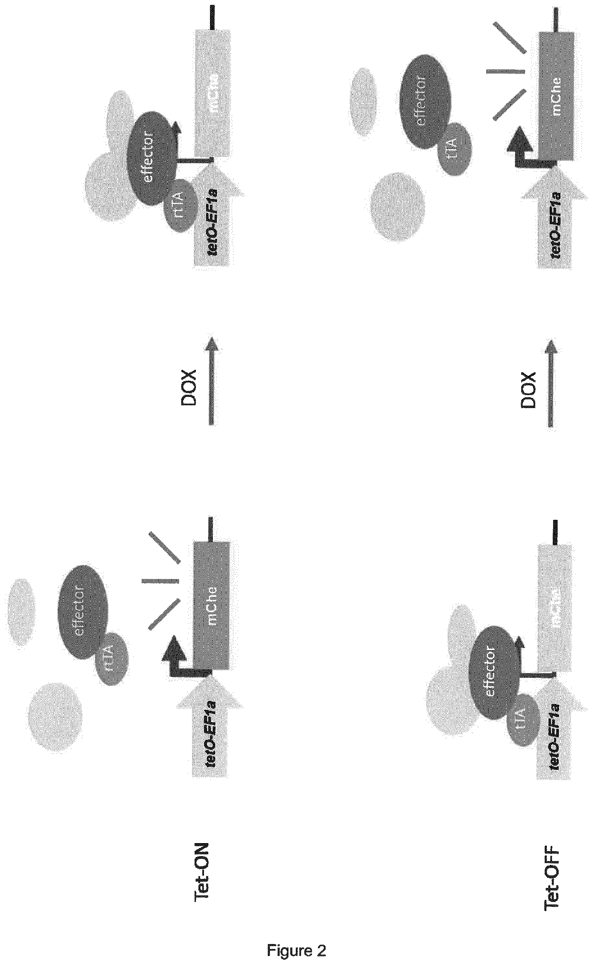

In step (a) of the methods of the present invention, a cell is provided that (i) expresses a fusion protein of said chromatin-associated protein with either a reverse tetracycline-controlled transactivator protein (rtTA) or a tetracycline-controlled transactivator protein (tTA), and (ii) is capable of expressing a reporter protein under control of a tetracycline response element (TRE) (Fig. 2). In this context, the use of the singular term "a cell" is not intended to imply that the methods of the present invention are to be performed on a single cell level. In particular, said methods usually are performed on a multi-cell level, i.e., they use a large, suitably selected number of cells at the same time.

Types of cells that can be used in the methods of the present invention are not particularly limited, are known in the art, and can be selected based on the specific points of interest to be addressed. Respective cells include for example cancer cell lines known in the art.

Specific examples of cell lines that can be of interest and can be used in the context of the present invention include the cell lines CL-52b, U2OS#18-XRCC2-5E/X2+, U2OS#18-RAD51D-4/D+, U2OS#18-RAD51C-15/C+, U2OS#18-RAD51B-8/B+, U2OS#18-XRCC3-6A, U2OS#18-XRCC2-5E, U2OS#18-RAD51D-4, U2OS#18-RAD51C-15, U2OS#18-RAD51B-8, U2OS#18, EL-4, CHP-100, JVE-367, MCA-B1, IPH-926, JVE-528, JVE-371, KMST-6-TNF, JVE-253, JVE-241, JVE-222, JVE-207, JVE-192, JVE-187, CX-03, MCC-60, MAPAC-HS-77, JVE-127, JVE-114, GH, CCC-5, JVE-109, KP-363T, JVE-103, JVE-059, 538-MG-BA, 170-MG-BA, JVE-017, GL-261, MBU-TS4, MBU-TS1, MBU-T, ETCC-016, ETCC-011, MBU-IM, U-2946, PCL-12, ETCC-010, ETCC-008, ETCC-007, ETCC-006, BLN-7, JIH-5, BLN-3, JVE-015, U-2-OS, FADU, PANC-1, NALM-33, HS-578T, HC-11, WSU-DLCL, LOPRA-1, MOLM-14, PG-EBV, UOC-M1, 232A4, CII, L4.5, CI, WA-C3CD5+, U-2904, WA-OSEL, PGA-1, HG-3, I83-LCL, I83-E95, HAIR-M, OCI-LY3, BXPC-3, LS-174T, SK-MM-1, BJAB, R-06E, MUG-CHOR-1, R-05T, PACADD-188, 1618-K, PACADD-183, PACADD-165, U-CH2, U-CH1, SUSA, PACADD-161, PACADD-159, JOPACA-1, GCT-27, BALL-1, 17.94, HD-MB03, T-47D, MDA-MB-468, NCI-H460, SK-BR-3, HT-93, CALU-6, MIA-PACA-2, MDA-MB-231, CAKI-1, OCI-AML6, OCI-AML4, MHH-TALL-2, P30-OHKUBO, OCI-AML1, KHYG-1, CARNAVAL, TK-6, OCI-LY1, NCEB-1, MOLM-1, MEGAL, MEG-A2, MAVER-1, LAZ-221, KASUMI-3, GRANTA-452, SK-GT-4, PACADD-137, PACADD-135, PACADD-119, ESO-26, HH, OE-33, IPEC-1, OAC-P4C, KYAE-1, SK-GT-2, IPEC-J2, OE-19, OCI-LY18, FLO-1, MKPL-1, OAC-M5.1, KI-JK, ESO-51, ELF-153, YNH-1, UCSD-AML1, SKNO-1, MUTZ-8, OCI-LY7, MINO, KASUMI-6, HD-MAR-2, BL-100, BCBL-1, NALM-21, NALM-20, NALM-16, BC-3, BC-2, BC-1, NGP, LS, LAN-6, LAN-5, UPCI-SCC-200, LAN-2, UPCI-SCC-090, UPCI-SCC-154, UPCI-SCC-131, UPCI-SCC-072, UPCI-SCC-111, UPCI-SCC-099, UPCI-SCC-074, UPCI-SCC-116, UPCI-SCC-114, UPCI-SCC-172, UPCI-SCC-040, UPCI-SCC-029A, UPCI-SCC-026, NMB, NBL-S, LAN-1, GI-ME-N, CHP-134, WILL-2, WILL-1, CRO-AP6, HCEC-H9C1, DND-39, HCEC-B4G12, HCEC-12, SHI-1, DOGUM, SH-2, U-2973, ST, PK-15, NT-92, MB-1, LLC-PK1, DT-40, 293T, U-2940, U-2932, SK-N-BE(2), HB-894, GUMBUS, DOGKIT, CL-49IV, ULA, U-HO1, BL-2, TA-85B5, CL-50IIa, CL-44, CL-38, FL-106, OCI-M2, SCC-4, SCC-25, FL-64, GF-D8, FKH-1, RL, WSU-FSCCL, MOLM-6, HAL-01, R3327-MATLyLu, SET-2, MOLP-2, KMS-12-PE, SUP-T11, RS-5, RAMOS, L-591, KYO-1, HNT-34, BEN-MEN-1, T2, L-82, JL-1, DM-3, BLUE-1, AP-1060, RH-41, MOLM-20, JIYOYE, JIMT-1, 8709, 7926, VAL, RI-1, REC-1, NU-DHL-1, OCI-AML3, HCT-116, HTC-C3, NU-DUL-1, TOM-1, SU-DHL-16, SU-DHL-10, WSU-DLCL2, SUP-HD1, SU-DHL-8, SU-DHL-6, SU-DHL-5, CI-1, MOLP-8, NCI-H510A, HT, HCC-827, C2C12, EN, HCC-78, RC-K8, EJM, MOTN-1, SC-1, ROS-50, NCI-H82, MOLM-16, MOLM-13, JEKO-1, KOPN-8, KMS-12-BM, C6, AML-193, RCH-ACV, SKM-1, SEM, H-2171, F-36P, NOMO-1, JJN-3, H-1963, DB, AMO-1, ME-1, PL-21, CL-40, HCC-44, JURL-MK2, JURL-MK1, DERL-2, L-1236, OCI-M1, OCI-LY19, NTERA-2, KASUMI-2, DND-41, DERL-7, SEWA, NALM-19, TALL-1, CL-34, KCL-22, SK-ES-1, HCC-1143, TC-71, CAL-29, HCC-1937, ARH-77, ALL-SIL, TT2609-C02, SUP-M2, "RS4;11", ONCO-DG-1, JMSU-1, CL-14, HCC-1599, H-1184, MEL-745A cl. DS19, MEC-2, H-209, KCI-MOH1, MEC-1, HCC-15, SU-DHL-4, HDQ-P1, RH-1, HCC-366, RH-18, MUTZ-5, RH-30, NK-92, HCC-33, PR-1, HH-16 cl.2/1, MMQ, HPB-ALL, GH4-C1, ACH1P, SOM-4D10, VIP-VIIIC8, OTH-74D4, CF-10H5, CF-1D12, CAT-13.9C1, CAT-13.1E10, CAT-13.0B10, BAG-85D10, BAG-12G2, GH3, SIG-M5, CL-11, RED-6, PC-3, ML-1, JEG-3, JAR, ESS-1, CAL-148, CAL-120, BEWO, AC-1M88, AC-1M81, AC-1M59, AC-1M46, WMP-2, RO, RED-5, BC-3C, CAL-78, CAL-62, CAL-33, CAL-27, HKT-1097, FU-OV-1, CAL-12T, AC-1M32, MS-5, CAL-85-1, CAL-72, 22RV1, MOLT-14, MOLT-13, HSB-2, YT, EVSA-T, 8-MG-BA, 42-MG-BA, SK-MM-2, VLM, D3, DU-4475, SW-1710, RED-4, 1G1, MFE-319, MFM-223, KG-1a, SER-W3, MFE-296, RT-112, HN, C7, RLD-1, 647-V, 639-V, RT-4, 32D, MFE-280, MKN-45, GOS-3, RED-1, U-2197, GMS-10, BHY, MT-3, RPMI-8226, FDCP-Mix cl.A4, VM-CUB1, TANOUE, BD-215, HT-1376, BE-13, KU-19-19, LOUCY, LOU-NH91, CMK, RMB-1, MSTO-211H, SUP-B15, LCLC-97TM1, KYSE-450, EOL-1, EGI-1, LCLC-103H, EPLC-272H, YAPC, KYSE-410, KYSE-270, KYSE-180, KU-812, TCC-SUP, T-24, KYSE-150, KYSE-510, SCLC-22H, SCLC-21H, KYSE-520, B-16V, SR-786, FDCP-1, BFTC-909, SD-1, CAL-54, MEG-01, KYSE-70, MOLT-4, BFTC-905, CGTH-W-1, DBTRG-05MG, HH-16.cl.4, HCT-15, SU-DHL-1, KELLY, MHH-PREB-1, SK-MES-1, RTG-2, KYSE-30, LOVO, LAT, KYSE-140, JK-1, HD-MY-Z, MH-7777A, TFK-1, SGE-1, GRANTA-519, MHH-CALL-2, M3E3/C3, MHH-CALL-3, DEL, MHH-CALL-4, MHEC5-T, V-79, TF-1, ST-2, RLC-18, M1, JTC-27, JTC-15, VA-ES-BJ, SISO, SW-948, SNB-19, BETA-TC-3, SR-4987, N2-261, SK-MEL-3, L-5178-Y, RAJI, N3-36, KPL-1, P-19, HT-1080, HPD-1NR, SW-480, RBL-2H3, EFM-192C, ECV-304, F1-652, EFM-192B, DV-90, CPC-N, 293, CHP-126, SK-MEL-1, CAL-51, HC-1, BA/F3, HT-29, BM-1604, A4-1077, A4-1025, MUTZ-3, SW-403, HPD-2NR, SF-158, U-138-MG, RPMI-8402, RV-C2, P-388D1(IL-1), RPMI-2650, TI-4, TI-1, PYS, JURKAT, 2A1, PAB-1620, BHT-101, DLD-1, DK-MG, A-2, CRO-AP3, BF-G6, B-CPAP, BF-32, MUTZ-2, LAMA-87, DMBM-2, C-433, AN3-CA, S-117, LXF-289, COLO-679, TE-671, RGE, DU-145, RD-ES, HUP-T3, EFM-192A, COLO-783, LNCAP, CADO-ES1, BEN, OMEGA-E, MONO-MAC-1, IPC-298, A-204, DAN-G, COLO-677, OCI-AML5, Y-79, CAPAN-2, CAPAN-1, SAOS-2, GAMG, PB-1, CCRF-CEM, IGR-39, PSI-2, IGR-37, IGR-1, EFO-21,A-427, BL-70, SC-75, EFM-19, EFE-184, SBC-7, SBC-2, BT-B, NUC-5, NUC-1, G/G, HUP-T4, HAP-T1, BF-45, KASUMI-1, 8505C, LCL-WEI, SC-71, COLO-849, CRO-AP5, A4-951, A4-840, BF-F3, B9, MC3T3-E1, SH-SY5Y, A-S-30D, NB-4, COLO-720L, 2M6, PA-TU-8988S, SK-N-MC, 3T6, 23132/87, COLO-824, NRK-52E, COLO-704, L-428, COLO-699, LF-CL2A, COLO-678, COLO-800, EBL, EFO-27, XTH-2, LN-405, PAB-100, CAL-39, SPC-BM-36, LCL-HO, D-36, 2HX-2, COLO-680N, IPL-LD-65Y, HEP-G2, PA-TU-8902, 2E10-H2, TN-368, BTI-EAA, HEPA 1-6, MDBK, 3T3, NRK-49F, BC3H1, J-774A.1, CACO-2, LAMA-84, MHH-ES-1, PAB-122, IMR-32, SIMA, NCI-H929, PA-TU-8988T, HELA-S3, BL-41, PC-12, KB-3-1, MHH-NB-11, BF-34, JOSK-I, BA-F8, FLK-BLV-044, AM-C6SC8, SK-MEL-30, BONNA-12, KB-V1, NEURO-2A, RBL-1, SP2/0-AG14, P3/NSI/1-AG4-1, COLO-320, BPH-1, SK-HEP-1, SUP-T1, PLB-985, MN-60, UT-7, KB, EM-2, EM-3, 8305C, A-10, NALM-1, SCHNEIDER-2, CX-1, NALM-6, RVH-421, CHO-DHFR[-], SF-9, MONO-MAC-6, L-1210, MB-04, GIRARDI HEART C7, NC-NC, SF-21, XC, IM-9, GIRARDI HEART C2, MCF-7, L138.8A, MB-L11, F9, IEC-6, CHO-K1, GM-7373, C6-BU-1, A-549, MH1C1, M-07e, N18TG2, MV4-11, N4TG3, B95-8, OCI-AML2, H25B10, MB-03, YAC-1, TMM, NS20Y, HEP-3B, SPI-802, A-431, WERI-RB-1, 72A1, THB-5, GDM-1, SPI-801, MB-021, MOLT-3, DG-75, MC-116, COLO-818, EB-1, MB-020, DAUDI, D-11, MB-L2, BA-D5, MEL-JUSO, CA-46, L-540, NAMALWA.IPN/45, NAMALWA.CSN/70, NAMALWA.PNT, NAMALWA.KN2, EHEB, RPMI-7951, MDA-MB-453, BT-474, COS-1, MEL-HO, BHK-21, COS-7, NIH-3T3, WSU-NHL, HELA, R1, A-498, CAKI-2, SKW-3, DA-1, OPM-2, L-363, CRO-AP2, DOHH-2, KE-37, D10.G4.1, ELM-I-1, X63AG8.653, 697, LP-1, CTV-1, 380, PF-382, KMOE-2, MOLT-17, 5637, P12-ICHIKAWA, VERO-B4, JOSK-M, MOLT-16, 4H1-A7, WEHI-3B, WEHI-164S, NAMALWA, 7-TD-1, REH, COLO-206F, BV-173, JVM-13, JVM-3, HDLM-2, THP-1, ML-2, KG-1, JVM-2, HEL, K-562, U-266, KM-H2, CML-T1, PEER, U-937, U-698-M, HL-60, L-929, and P-815, as known in the art.

The cells provided in step (a) of the methods of the present invention express a fusion protein of said chromatin-associated protein with either a reverse tetracycline-controlled transactivator protein (rtTA) or a tetracycline-controlled transactivator protein (tTA), i.e., said fusion protein is either a fusion protein of said chromatin-associated protein with rtTA, or a fusion protein of said chromatin-associated protein with tTA, wherein a fusion protein of said chromatin-associated protein with rtTA is preferred (Fig. 2). That is, the methods of the present invention either (i) use the TET-On system as known in the art, wherein in the absence of a tetracycline, a fusion protein of said chromatin-associated protein with rtTA is not associated with a *TetO* operator sequence, and in the presence of a tetracycline, said fusion protein is recruited to a *TetO* operator sequence, or (ii) use the TET-Off system as known in the art, wherein in the absence of a tetracycline, a fusion protein of said chromatin-associated protein with tTA is recruited to a *TetO* operator sequence, and in the presence of a tetracycline, said fusion protein is released from said *TetO* operator sequence (Fig. 2).

Means for providing cells expressing a fusion protein as defined above are not particularly limited and are known in the art. In specific embodiments, the cell provided in step (a) of the methods of the present invention has been transduced with a genetic element encoding said fusion protein under control of a suitable promoter. Respective methods for transducing cells, as well as suitable promoters, are not particularly limited and are known in the art. In a particular example, the cell can be transduced using viral, e.g. lentiviral, vectors as known in the art.

Further, the cells provided in step (a) of the methods of the present invention are capable of expressing a reporter protein under control of a tetracycline response element (TRE). Suitable reporter proteins that can be used in the methods of the present invention are not particularly limited and are known in the art. They include any proteins that generate a detectable signal, preferably a fluorescence signal. Further, suitable reporter proteins include any membrane-bound proteins, such as e.g. Thy1.1, that can be detected using antibodies that are labeled with a detectable label. Respective fluorescent proteins are not particularly limited and are known in the art.

In particular, fluorescent proteins can be selected from the group consisting of mCherry and green fluorescent protein (GFP). Further suitable fluorescent proteins include TagBFP, mTagBFP2, Azurite, EBFP2, mKalama1, Sirius, Sapphire, T-Sapphire, ECFP, Cerulean, SCFP3A, mTurquoise, mTurquoise2, monomeric Midoriishi-Cyan, TagCFP, mTFP1, EGFP, Emerald, Superfolder GFP, Monomeric Azami Green, TagGFP2, mUKG, mWasabi, Clover, mNeonGreen, EYFP, Citrine, Venus, SYFP2, TagYFP, Monomeric Kusabira-Orange, mKOκ, mKO2, mOrange, mOrange2, mRaspberry, mCherry, mStrawberry, mTangerine, tdTomato, TagRFP, TagRFP-T, mApple, mRuby, mRuby2, mPlum, HcRed-Tandem, mKate2, mNeptune, NirFP, TagRFP657, IFP1.4, iRFP, mKeima Red, LSS-mKate1, LSS-mKate2, mBeRFP, PA-GFP, PAmCherry1, PATagRFP, Kaede (green), Kaede (red), KikGR1 (green), KikGR1 (red), PS-CFP2, PS-CFP2, mEos2 (green), mEos2 (red), mEos3.2 (green), mEos3.2 (red), PSmOrange, PSmOrange, Dronpa, as known in the art.

Suitable TREs to be used in the methods of the present invention are known in the art. TREs can comprise a suitable promoter and one or more *TetO* operator sequences upstream of said promoter. In this context, suitable promoters are not particularly limited and are known in the art. They include the promoters CMV, EF1a, SV40, PGK1 (human or mouse), Ubc, human beta actin, CAG, TRE, UAS, Ac5, Polyhedrin, CaMKIIa, GAL1, 10, TEF1, GDS, ADH1, CaMV35S, Ubi, H1, U6, SFFV, MYCmin, as known in the art.

Means for providing cells that are capable of expressing a reporter protein under control of a TRE are known in the art. In specific embodiments, the cell provided in step (a) of the methods of the present invention has been transduced with a genetic element encoding said reporter protein under control of a suitable TRE. In preferred embodiments, the genetic element encoding said reported protein under control of said TRE is stably integrated into the genome of the cell. Respective methods for transducing cells, stably integrating a genetic element into the genome of the cell, as well as suitable TREs, are not particularly limited and are known in the art. In a particular example, the cell can be transduced using viral, e.g. lentiviral, vectors as known in the art.

In step (b) of the methods of the present invention, the expression of a factor of interest is modulated. In this context, the term "modulating the expression" is intended to refer to any up- or downregulation of the expression level of the factor of interest. The factor of interest, preferably protein factor of interest, is not particularly limited and includes any factor that is suspected of having a co-regulatory activity on a given chromatin-associated protein and/or a complex partner of said chromatin-associated protein, i.e., that is suspected of directly or indirectly modulating the activity of said given chromatin-associated protein, and/or a binding partner of said chromatin-associated protein, and/or a factor that modulates the activity of said chromatin-associated protein without forming a physically stable complex with the same.

Means for modulating the expression of a factor of interest are not particularly limited and are known in the art. They include RNA interference (RNAi) techniques and gene editing techniques using the CRISPR/Cas system, as known in the art. Briefly, RNAi techniques include any techniques known in the art using RNA molecules for inhibiting gene expression and/or translation by neutralizing targeted mRNA molecules. Further, gene editing techniques using the CRISPR/Cas system include any techniques known in the art using a Cas9 nuclease and guide RNAs (gRNAs) for the specific editing of a target genomic region. Additionally, gene editing techniques using the CRISPR/Cas system include the application of CRISPR interference (CRISPRi) or CRISPR activation (CRISPRa), *i.e.,* genetic perturbation techniques that allow for sequence-specific repression or activation of gene expression in eukaryotic cells at the DNA level without inflicting irreversible genetic alterations.

In specific embodiments, RNAi techniques include the use of RNAi libraries, i.e., collections of a multitude of different RNAi agents that modulate the expression of a multitude of factors of interest in a multitude of parallel instances of the methods of the present invention. Likewise, in specific embodiments, gene editing techniques using the CRISPR/Cas system include the use of gRNA libraries, *i.e.,* collections of a multitude of different gRNAs that allow the editing of a multitude of genes encoding a multitude of factors of interest in a multitude of parallel instances of the methods of the present invention.

In step (c) of the methods of the present invention, a tetracycline is provided to the cell in amount sufficient to induce binding of rtTA to the TRE or release of tTA from the TRE while the modulation of the expression of said factor of interest persists, *i.e.,* while an up- or downregulation of the expression level of said factor of interest is still in effect. In preferred embodiments, the tetracycline is doxycycline. Typically, the tetracycline is provided to the cells by addition of the same to the culture medium. Suitable concentrations of the tetracycline, e.g. of doxycycline, are in the range of 0.1 to 2 µg/ml in the culture medium, preferably 0.5 to 1 µg/ml in the culture medium.

In step (d) of the methods of the present invention, any modulation of the expression of the reporter protein, *i.e.,* any up- or down-regulation of the expression level of said reporter protein, is detected. Means for detecting such modulation for any given kind of reporter protein are not particularly limited and are known in the art. In the case of fluorescent proteins or membrane reporter proteins that are detected using fluorescent antibodies, said modulation can be detected by way of fluorescence activated cell sorting (FACS) techniques or fluorescence microscopy.

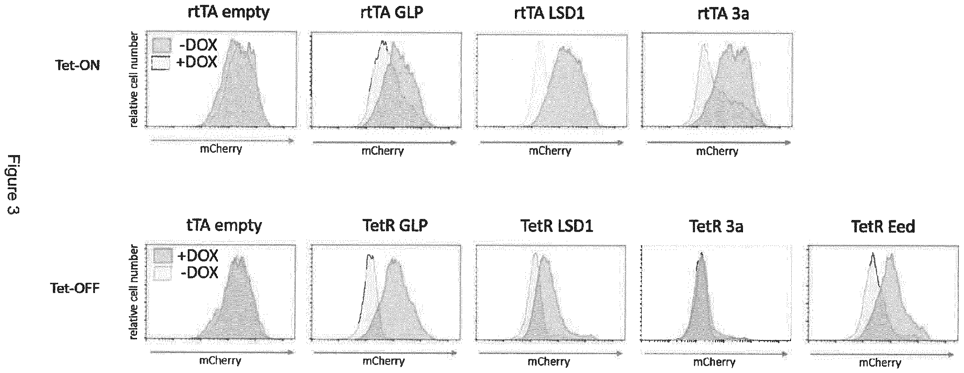

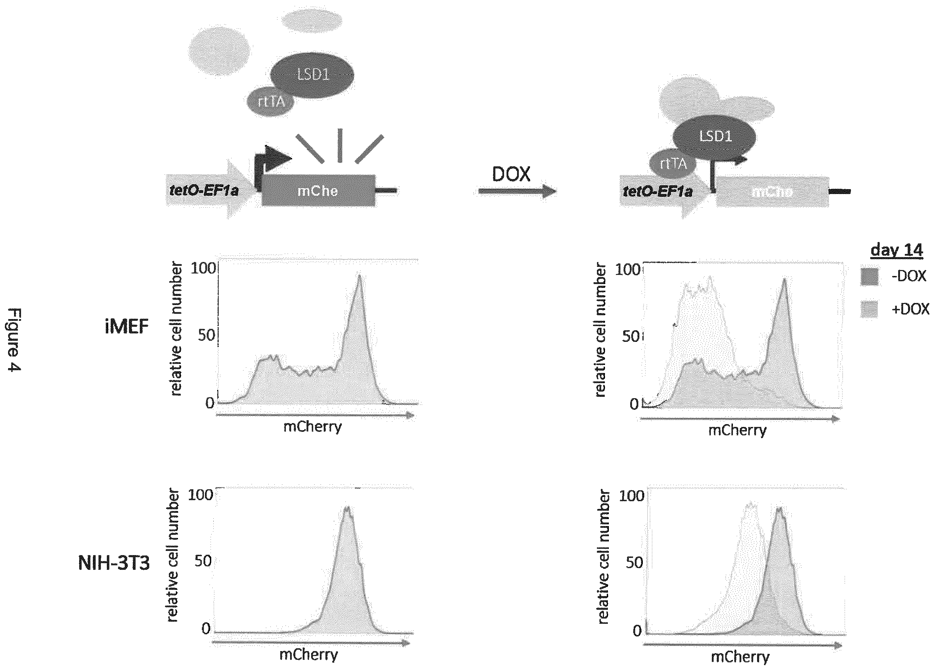

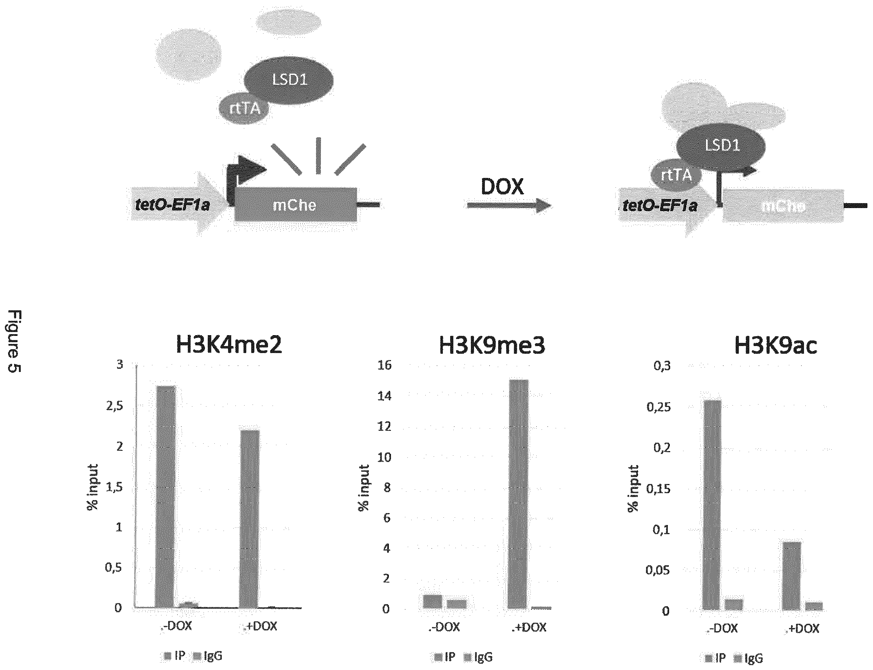

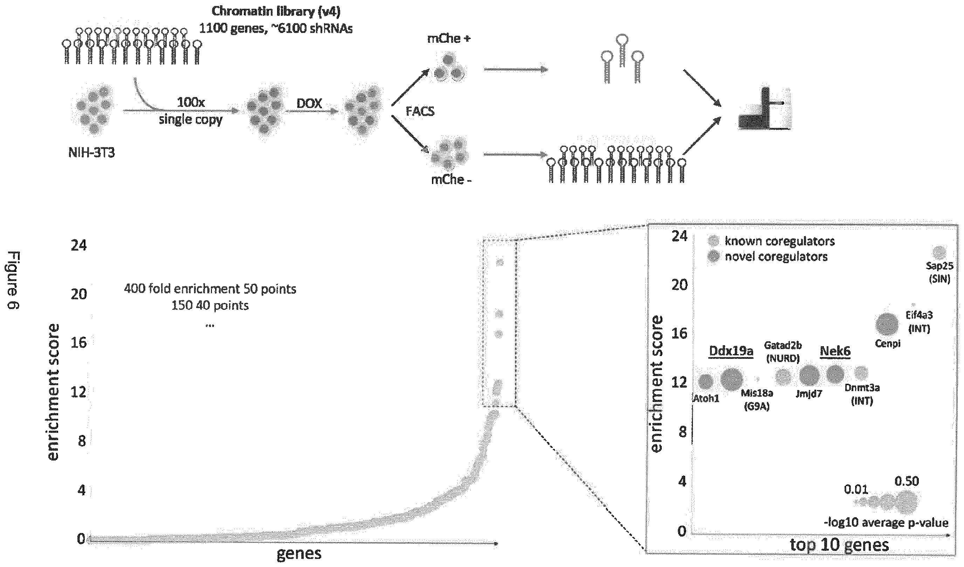

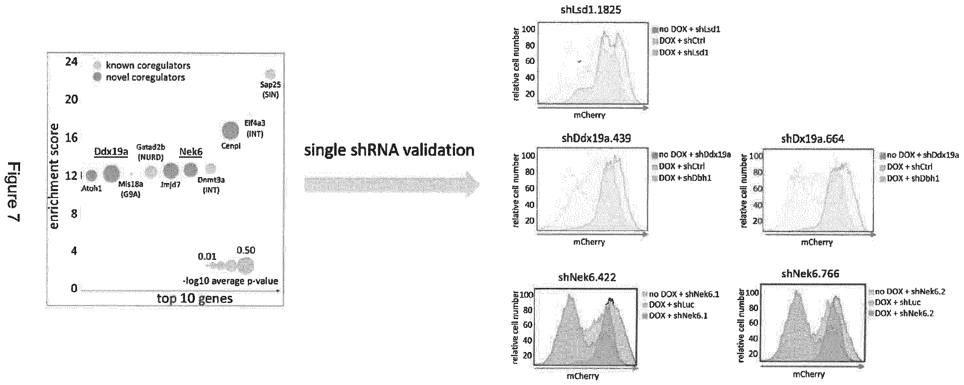

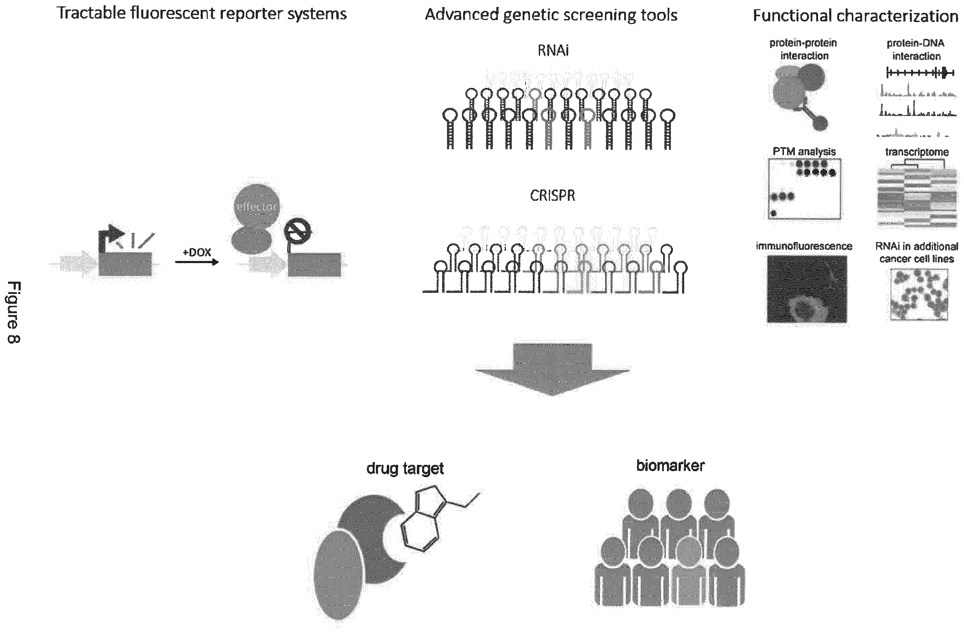

In step (e) of the methods of the present invention, the factor of interest whose expression has been modulated in step (b) is identified as a co-regulatory factor that modulates the activity of said chromatin-associated protein and/or a binding partner of said chromatin-associated protein, and/or a factor that modulates the activity of said chromatin-associated protein without forming a physically stable complex with the same, in case the modulation of the expression of said reporter protein detected in step (d) differs from the modulation of the expression of said reporter protein detected in a control cell in which the expression of said factor of interest has not been modulated (Fig. 3). Thus, according to the present invention, any modulation of the expression of the reporter protein detected in step (d) of the methods of the present invention has to be correlated to the modulation of the expression of the reporter protein in a control cell which has been subjected to the same methods but without any modulation of the expression of the factor of interest in the control cell, wherein any difference is the respective modulations indicates a co-regulatory activity of the factor of interest (Fig 3).

In preferred embodiments, the methods of the present invention are performed *in vitro, i.e.,* they are not performed on the human or animal body.

As used herein, the term "comprises/comprising" is expressly intended to encompass the terms "consists essentially of/consisting essentially of" and "consists of/consisting of".

In view of the complexity of epigenetic mechanisms and their regulation (Fig. 1), it is important to significantly increase the level of knowledge in order to develop possible therapeutic approaches. In order to investigate the specific function of epigenetic proteins and to directly analyze the complex regulatory network, a suitable reporter system was developed in which the expression of a fluorescent reporter gene in the cell is controlled by the recruitment of epigenetic effectors. This allows the functional and hypothesis-free characterization of chromatin networks and effector protein-associated multiprotein complexes and the identification of essential components (proteins or protein complexes) that influence the activity of the epigenetic effector and the stability of the induced chromatin modifications.

The tetracycline repressor system (Tet system) was used as the basis for the novel epi reporter assays according to the present invention. Epigenetic effector proteins are amplified from cDNA or obtained by gene synthesis and expressed as fusion protein with the Tet repressor or reverse Tet repressor protein in cell lines. These cell lines simultaneously possess a fluorescence reporter gene (mCherry/GFP, etc.) which is stably integrated into the genome and which has a certain number of Tet repressor binding sites (*TetO*) upstream of the promoter. The addition of doxycycline recruits the epi effector together with its associated complex partners and co-regulators to the Tet repressor binding sites and influences the expression of the fluorescence gene. The change in expression is a direct consequence of the recruitment of the complex, which alters the modifications of chromatin to the promoter of the fluorescence gene (Fig. 2).

This makes it possible to study the activity of an effector together with its complex. This allows the hypothesis-free analysis of the complex partners in view of their importance for the activity of the complex and enables new target proteins for drug development to be identified via genetic screens or high-throughput inhibitor screens (Fig. 3).

The methods of the present invention advantageously do not require the purification of proteins or protein complexes, and do not the generation of inhibitor derivatives via extensive chemical synthesis pathways that are used for "chemoproteomics". Epigenetic effectors can be amplified from cDNA with specific primers or generated by gene synthesis. Further, expression in all common cell lines via viral transduction is possible. After transduction into the cell line, the epi effector is recruited with all cell line specific complex partners and co-regulators via the addition of doxycycline to the fluorescence reporter and regulates the fluorescence expression, which can be quantified by flow cytometry or fluorescence microscopy (Fig. 3). Thus, inhibitor screens or functional genetic screens (CRISPR/RNAi) can be used to identify new drug targets that would not be identified by original assays.

The figures show:
Figure 1:
   Schematic representation of the complex regulatory epigenetic network. Posttranslational modifications at the DNA and histone proteins regulate gene expression through the coordinated recruitment of coregulator complexes. The transcriptional output of the recruited effector proteins (e.g. epigenetic effectors, transcription factors or nuclear receptors) is determined by a highly complex network of additional coregulators. Our limited mechanistic understanding of most of these coregulators complicates the understanding of their function in different cancer contexts. Concurrently, the dependence of effector proteins on accessory proteins gives various opportunities for therapeutic intervention.
Figure 2:
   Schematic representation of the Tet-ON and Tet-OFF reporter systems. The effector protein is expressed as a rtTA or tTA fusion protein. Addition of tetracycline (here: DOX) results in binding of the rtTA-effector fusion protein to the tetO promoter (Tet-ON) or release of the tTA-effector fusion protein from the promoter (Tet-OFF), which results in altered expression of the reporter protein (mCherry).
Figure 3:
   Flow cytometry profiles depicting the expression of the reporter fluorophore in the presence of different rtTA- and tTA-effector fusion proteins. Top: rtTA-fusion proteins without (red) and with (grey) DOX treatment. Bottom: tTA-fusion proteins without (grey) and with (red) DOX treatment and rtTA/tTA without any effector protein (-empty) serves as a control. DOX addition to the culture medium results in effector-specific changes in reporter protein (mCherry) expression independently of the fusion partner rtTA or tTA.
Figure 4:
   Flow cytometry profiles depicting the expression of the reporter fluorophore in presence of a rtTA-LSD1 fusion protein before (red) and with (grey) DOX treatment in two mouse fibroblast cell lines. DOX addition to the culture medium results in cell-type specific changes in reporter protein (mCherry) expression.
Figure 5:
   Changes of histone modifications at the reporter in response to LSD1 recruitment. Chromatin immunoprecipitation (ChIP) followed by qPCR shows a reduction of histone H3 lysine 4 dimethylation (LSD1 catalytic activity) and effects resulting from recruitment of known coregulators of LSD1, e.g. G9a/Glp (H3K9me3) and HDACs (H3K9ac).
Figure 6:
   Outline of the multiplexed RNAi screening strategy. Engineered reporter cell lines are transduced with shRNAs (coupled to GFP expression) prior to recruitment of LSD1 with DOX. After several passages, cells expressing effective shRNAs, identified via FACS by the remaining reporter fluorescence (mChe+), are separated from cells expressing ineffective shRNAs, indicated by reduced expression of the fluorescent reporter protein (mChe-). Genomic DNA is extracted from both populations and the abundance of shRNAs is assessed by deep-sequencing of the guide sequences. The abundance of shRNAs found in the mChe+ compared to the abundance in the mChe- population is allocated to an enrichment score for each gene, summarizing the enrichments of all shRNAs per gene. Scatter plot depicting all genes included in the library ranked by average median enrichment of all shRNAs across five technical replicates. The 10 most important LSD1 coregulators identified are highlighted. grey = known interactors, red = novel coregulators of LSD1. Dot size indicates respective p values (n = 5). Several proteins associated with the G9a (Mis18a), NURD (Gatad2b) and SIN (Sap25) complexes were identified as being important for LSD1-mediated silencing. Among several other novel and interesting dependencies, the screen identified the ATP-dependent RNA helicase Ddx19a and the NIMA-related kinase Nek6 as novel coregulators with highest significance.
Figure 7:
   Single shRNA validation of selected hits. Transduction of reporter cell lines with two shRNAs for each Ddx19a or Nek6 in individual experiments reproduce the findings observed in the screen. Reduction of Ddx19a as well as Nek6 negatively influenced LSD1 silencing activity on the reporter gene, compared to silencing in cell lines expressing a control shRNA (shLuc). shRNA-targeting of the rtTA-LSD1 fusion construct with shLSD1.1825 serves as a positive control.
Figure 8:
   Tractable fluorescence reporter systems allow to identify novel critical coregulators of epigenetic effectors using functional genetic screens. Following primary validation of identified coregulators, different methods can be employed to functionally characterize the mechanism by which each candidate interferes with the effector function. The results will provide a basis for the identification of novel potential drug targets in cancer therapy and establish biomarkers for personalized medicine.

The present invention will be further illustrated by the following example without being limited thereto.

### Examples

### Material and methods:

### Reporter design

All reporter vectors were created as plasmids under the control of an EF1 a promoter driving the fluorescent reporter gene (mCherry) and an antibiotic resistance (e.g. Blasticidin (Invitrogen, R210-01)) coupled via a P2A sequence. Six tetO binding sites were synthetized and introduced upstream of the EF1a promoter using restriction site cloning to generate the TECB6 reporter construct.

### rtTA/tTA effector fusion constructs

For the rtTA and tTA effector fusions, genes of interest were PCR amplified from cDNA and inserted into the delivery constructs by Gibson assembly.

### Cell line transduction: TECB6 construct (MSCV)

Retroviral packaging was performed using Platinium-E cells (Cell Biolabs) according to established protocols. In brief, for each calcium phosphate transfection, 10-20 µg plasmid DNA and 5 µg helper plasmid (pCMV-Gag-Pol, Cell Biolabs) were used. Transduction efficiencies of retroviral constructs were measured 48 h post infection by flow cytometry (MACS VYB, Miltenyi). Transduced cell populations were selected starting 48 h post infection using 10 µg ml⁻¹ Blasticidine for 5 days.

### rtTA/tTA effector fusion

Lentivirus was produced by PEI co-transfection of the desired construct and two packaging vectors VSV-G (addgene #8454) and pCMVR8.74 (addgene # 22036) in HEK293T cells. After 48-72 hours the virus was collected. NIH-3T3 or iMEFs were then transduced with the virus for 48 hours in the presence of 8 mg/ml polybrene (Santa Cruz Biotechnology, SACSC-134220). Transduced cell populations were selected starting 48 h post infection using 500 µg ml⁻¹ Hygromycine for 5 days.

### Cell culture conditions

Packaging cell lines (HEK-293T and PlatE) as well as murine fibroblast cell lines (NIH-3T3 and iMEF) were cultured in DMEM (Gibco-Invitrogen) supplemented with 10% FBS, 20 mM glutamate, 10 mM sodium pyruvate, 100 U ml⁻¹ penicillin and 100 µg ml⁻¹ streptomycin.

### Flow cytometry analysis and sorting

All flow cytometry analyses were conducted on a MACS VYB (Miltenyi Biotech) using FlowJo software. For fluorescent cell sorting a FACS ARIA III (BD Biosciences) or a SH800S (Sony) was used.

### Chromatin Immunoprecipitation (ChIP-qPCR)

2×10⁶ cells per ChIP were harvested and crosslinked with 1% formaldehyde for 5 min. Crosslinking was stopped with 250 mM glycine and cells were washed with 10 ml ice-cold PBS. Cells were lysed with 1 ml cell lysis buffer (10 mM Tris pH 8, 10 mM NaCl, 0.2% NP-40, EDTA-free protease inhibitor), nuclei were pelleted down and lysed in 350 µl nuclear lysis buffer (50 mM Tris pH 8, 10 mM EDTA, 1% SDS, EDTA-free protease inhibitor) for 30 min at room temperature. Chromatin was fragmented to a size of 100-500 bp by sonication. For pre-clearing, chromatin was incubated in 3.5 ml IP dilution buffer (20 mM Tris pH 8, 2 mM EDTA, 150 mM NaCl, 1% TritonX-100, 0.01 % SDS, EDTA-free protease inhibitor) with 2.5 µg rabbit IgG and 25 µl Protein G Dynabeads (ThermoFisher) for 2 h at 4°C. Beads were locked with a magnet and the supernatant was transferred to a fresh tube. 180 µl were taken for Input and the rest was split in two. For IP, 2.5 µg of H3K9me3 (ab8898, Abcam), H3K9ac (AH3-120, ab12179, Abcam), H3K4me2 (ab7766, Abcam) or rabbit/mouse IgG were added to the solution and rotated over night at 4°C. 25 µl of Protein G Dynabeads were added and incubated at 4°C for 2 h. Beads were washed once with 1 ml IP wash1 (20 mM Tris pH8, 2mM EDTA, 50 mM NaCl, 1% TritonX-100, 0.1 % SDS), twice with high salt buffer (20 mM Tris pH 8, 2mM EDTA, 500 mM NaCl, 1% TritonX-100, 0.01 % SDS), once with IP wash2 (10 mM Tris pH 8, 1 mM EDTA, 0.25 M LiCI, 1% NP-40, 1% deoxycholic acid) and twice with TE buffer (10mM Tris pH 8, 1 mM EDTA). DNA was eluted in two steps à 100 µl in 1% SDS, 100mM NaHCO₃ for 30 min at room temperature. NaCl was added to a final concentration of 250 mM and IPs and inputs were digested with 10 ng/µl RNAse A at 65°C over night. Remaining proteins were digested with 0.25 mg/ml Proteinase K for 2h at 45°C. Clean up of DNA was done with chromatin purification kit (Active Motif). Pulldown efficiency relative to input was determined with quantitative PCR (ORA™ SEE qPCR Green ROX L Mix, highQu).

### Example: Functional genetic screen to identify novel co-regulators of LSD1

### shRNA library construction

The shRNA library used in the screen was cloned into pSGEN (pRRL-SFFV-GFP-miRE-PGK-NeoR, Addgene #111171). The same vector was used for validation of primary hits and PRC2 core complex partners.

### shRNA library screen

An shRNA library targeting 1100 chromatin-associated mouse genes was assembled in pSGEN, cloned and pooled. After spiking in several control shRNAs at equimolar ratios, the final pool of 6600 SGEN-shRNAs was transduced in five replicates into NIH-3T3 cells harboring a genomic integration of the TECB6 reporter and constitutively expressing the rtTA-LSD1 fusion construct. To ensure library representation, a total of 80 million cells were infected with 10% transduction efficiency, using conditions that predominantly lead to a single retroviral integration per cell and represent each shRNA in a calculated number of > 1000 cells. Throughout G418 drug selection (2.5 mg ml⁻¹), more than 50 million cells were maintained at each passage to preserve library representation. Seven days after beginning of G418 selection, binding of the rtTA-LSD1 fusion protein was induced by the addition of 1µg/ml Doxycycline (DOX). After seven days of DOX treatment, mCherry positive cells were separated from mCherry negative cells by FACS sorting for each replicate.

Genomic DNA from mCherry positive and negative samples was isolated two rounds of phenol extraction using PhaseLock tubes (5PRIME), followed by ethanol precipitation. Deep-sequencing libraries were generated by a two-step PCR amplification of shRNA guide strands using primers that tag the product with standard Illumina adapters (p7+Loop: CAAGCAGAAGACGGCATACGATAGTG AAGCCACAGATGT (SEQ ID NO: 1); p5+PGK: AATGATACGGCGACCACCGA TGGATGTGGAATGTGTGCGAGG (SEQ ID NO: 2)). For each sample, DNA from at least 5 × 10⁶ cells was used as template in multiple parallel 50-µl PCR reactions, each containing 0.5 µg template, 1 × AmpliTaq Gold buffer, 0.2 mM of each dNTP, 2 mM MgCl₂, 0.3 µM of each primer and 2.5 U AmpliTaq Gold (Life Technologies), which were run using the following cycling parameters: 95 °C for 10 min; 35 cycles of 95 °C for 30 s, 52 °C for 45 s and 72 °C for 60 s; 72 °C for 7 min. PCR products (340 bp) were combined for each sample, column purified using the QIAquick PCR purification kit (Qiagen) and further purified on a 1% agarose gel (QIAquick gel extraction kit, Qiagen). Libraries were analyzed on an Illumina HiSeq 2000 deep sequencer; 22 nucleotides of the guide strand were sequenced using standard illumina sequencing primer. To provide a sufficient baseline for detecting shRNA depletion in experimental samples, it was aimed to acquire >500 reads per shRNA in the sequenced shRNA pool to compensate for variation in shRNA representation inherent in the pooled plasmid preparation or introduced by PCR biases. With these conditions, baselines of > 500 reads for all 6600 shRNAs were acquired. Sequence processing was performed using a customized Galaxy platform. For each shRNA and condition, the number of matching reads was normalized to the total number of library-specific reads per lane and imported into a database for further analysis (EXCEL 2016, Microsoft). Afterwards, the enrichment score for each gene was generated by calculating the ratio of the geometric mean of all replicates of the mCherry positive and mCherry negative population for each shRNA and summarizing all shRNAs for each gene.

### Validation of screening hits

NIH-3T3 cells harboring a genomic integration of the TECB6 reporter and constitutively expressing the rtTA-LSD1 fusion construct were transduced with pSGEN (vectors harboring the respective shRNAs targeting known or novel coregulators of LSD1 at infection rates of <10% to ensure single-copy integration. Transduced cells were selected using G418 (2.5 mg ml⁻¹) for 7-21 days before the addition of DOX to induce recruitment of the rtTA-LSD1 fusion construct to the TECB6 reporter construct. Fluorescence (mCherry) was monitored for 14-21 days by flow cytometry.

## Claims

1. A method for the identification of a co-regulatory factor that modulates the activity of (i) a chromatin-associated protein and/or (ii) a binding partner of said chromatin-associated protein, and/or (iii) a factor that modulates the activity of said chromatin-associated protein without forming a physically stable complex with the same, in a cell, comprising the steps of:
(a) providing a cell that
(i) expresses a fusion protein of said chromatin-associated protein with either a reverse tetracycline-controlled transactivator protein (rtTA) or a tetracycline-controlled transactivator protein (tTA), and
(ii) is capable of expressing a reporter protein under control of a tetracycline response element (TRE);
(b) modulating the expression of a factor of interest in said cell;
(c) providing a tetracycline to the cell in amount sufficient to activate rtTA or tTA while the modulation of the expression of said factor of interest persists;
(d) detecting any modulation of the expression of said reporter protein in said cell;
(e) identifying the factor of interest as a co-regulatory factor that modulates the activity of said chromatin-associated protein and/or a binding partner of said chromatin-associated protein, and/or a factor that modulates the activity of said chromatin-associated protein without forming a physically stable complex with the same, in case the modulation of the expression of said reporter protein detected in step (d) differs from the modulation of the expression of said reporter protein detected in a control cell in which the expression of said factor of interest has not been modulated.

2. The method according to claim 1, wherein the chromatin-associated protein is an epigenetic effector protein.

3. The method according to claim 2, wherein the epigenetic effector protein is selected from the group, consisting of Lysine-specific histone demethylase 1A (LSD1),), Euchromatic Histone Lysine Methyltransferase 1 (EHMT1), DNA Methyltransferase 3 Alpha (DNMT3A) and Embryonic Ectoderm Development (EED).

4. The method according to any one of claims 1 to 3, wherein the cell provided in step (a) has been transduced with a genetic element encoding said fusion protein under control of a suitable promoter.

5. The method according to any one of claims 1 to 4, wherein the reporter protein is selected from the group consisting of mCherry and green fluorescent protein (GFP).

6. The method according to any one of claims 1 to 5, wherein the TRE comprises a suitable promoter and one or more *TetO* operator sequences upstream of said promoter.

7. The method according to any one of claims 1 to 6, wherein the genetic element encoding said reported protein under control of said TRE is stably integrated into the genome of the cell.

8. The method according to any one of claims 1 to 7, wherein the factor of interest is a protein.

9. The method according to any one of claims 1 to 8, wherein modulation of the expression of said factor of interest in step (b) is achieved via RNA interference (RNAi) techniques or via gene editing techniques using the CRISPR/Cas system.

10. The method according to any one of claims 1 to 9, wherein the tetracycline provided to the cell in step (c) is doxycycline.

11. The method according to any one of claims 1 to 10, wherein detection of any modulation of the expression of said reporter protein in step (d) is achieved via fluorescence activated cell sorting (FACS) techniques or via fluorescence microscopy.

12. The method according to any one of claims 1 to 11, wherein the cell provided in step (a) expresses a fusion protein of said chromatin-associated protein with rtTA.

13. The method according to any one of claims 1 to 12 which is performed *in vitro.*


SEQUENCE LISTING

<110>  Universität Stuttgart

<120>  Fluorescence-based cellular assay for the detection of
       gene-regulatory activity of chromatin-associated proteins and
       protein complexes

<130>  S14434EU - kl

<160>  2

<170>  PatentIn version 3.5

<210>  1
<211>  39
<212>  DNA
<213>  Artificial sequence

<220>
<223>  PCR primer

<400>  1
caagcagaag acggcatacg atagtgaagc cacagatgt                              39


<210>  2
<211>  42
<212>  DNA
<213>  Artificial sequence

<220>
<223>  PCR primer

<400>  2
aatgatacgg cgaccaccga tggatgtgga atgtgtgcga gg                          42